# EUROPEAN PATENT SPECIFICATION

(11) **EP 0 652 708 B1**
(45) Date of publication and mention of the grant of the patent: **17.04.1996**
(21) Application number: 93915950.5
(22) Date of filing: 19.07.1993
(51) Int. Cl.: A01N 47/36, A01N 25/32

(54) **SELECTIVE HERBICIDAL COMPOSITION**
Selektive herbizide Zusammensetzung
COMPOSITION HERBICIDE SELECTIVE

(30) Priority: 30.07.1992 CH 2401/92
(43) Date of publication of application: 17.05.1995
(73) Proprietor: CIBA-GEIGY AG, CH-4002 Basel (CH)
(72) Inventor: GLOCK, Jutta, CH-4336 Kaisten (CH); KERBER, Elmar, D-7883 Görwihl (DE); FÖRY, Werner, CH-4125 Riehen (CH); SCHURTER, Rolf, CH-4102 Binningen (CH)
(86) International application number: EP9301898
(87) International publication number: WO9403064

(56) References cited:
- EP-A- 0 103 543
- EP-A- 0 492 367
- EP-A- 0 582 021

## Description

The present invention relates to a selective herbicidal composition for controlling grasses and weeds in crops of cultivated plants, especially in cereals crops, which composition consists of a herbicide and a safener (antidote) that protects the cultivated plants, but not the weeds, from the phytotoxic action of the herbicide, and to the use of said composition or combination of herbicide and safener for controlling weeds in crops of cultivated plants.

When applying herbicides, crop plants may be severely damaged owing to such factors as concentration of herbicide and type of application, species of crop plant, soil characteristics, temperature and rainfall. In particular, severe damage may be caused when, in the course of crop rotation, other crop plants which have no or only insufficient resistance to herbicides are grown in succession to plants that are resistant to herbicides.

To counteract this problem, the proposal has already been made to use different compounds which are able specifically to antagonise the harmful action of the herbicide on the crop plant, i.e. to protect the crop plant without markedly influencing the herbicidal action on the weeds to be controlled. It has, however, been found that the proposed safeners very often have a species-specific activity both with respect to the crop plants and to the herbicide and, in some cases also, contingent on the mode of application, i.e. a specific safener is often suitable only for a specific crop plant and a specific class of herbicide. For example, EP-A-0 094 349 discloses quinoline derivatives that protect crop plants from the phytotoxic action of herbicides of specific compound classes, including phenoxy-propionate herbicides, ureas, carbamates or diphenyl ethers.

It has now been found that very specific quinoline derivatives disclosed in EP-A-0 094 349 are suitable for protecting crop plants from the phytotoxic action of a special class of sulfonyl urea herbicides.

Accordingly, the invention relates to a selective herbicidal composition which contains, in addition to inert components such as carriers, solvents and wetting agents, as active component a mixture comprising
a) a herbicidally effective amount of a sulfonyl urea of formula I 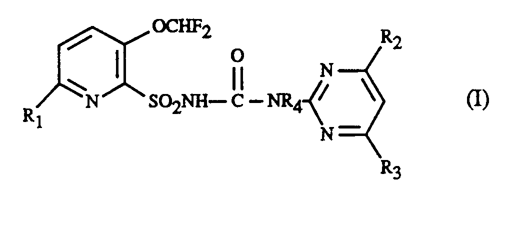 wherein
   R₁ is hydrogen or methyl;
   R₂ and R₃ are each independently of the other methyl or methoxy; and
   R₄ is hydrogen or methyl, or a salt of this compound with an amine, an alkali metal base or alkaline earth metal base or with a quaternary ammonium base; and
b) an effective amount of a quinoline derivative of formula II 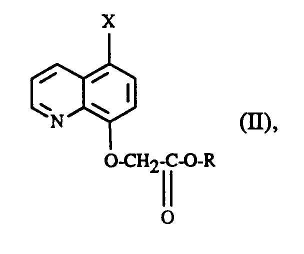 wherein
   R is hydrogen or C₁-C₈alkyl, and
   X is hydrogen or chlorine,
   as safener for antagonising the herbicide.

Suitable alkyl groups are straight-chain or branched alkyl groups, typically methyl, ethyl, n-propyl, isopropyl, n-butyl, isobutyl, sec-butyl or tert-butyl, as well as the different isomers of pentyl, hexyl, heptyl and octyl.

The compounds of formula I can form salts in which the hydrogen atom of the -SO₂-NH-group is replaced by an agriculturally suitable cation. These salts are typically metal salts, preferably alkali metal or alkaline earth metal salts, or also ammonium salts or salts with organic amines.

Illustrative examples of amines suitable for forming ammonium cations are primary, secondary and tertiary C₁-C₄alkylamines, C₁-C₄hydroxyalkylamines and C₂-C₄alkoxyalkylamines typically methylamine, ethylamine, n-propylamine, isopropylamine, the four isomeric butylamines, n-amylamine, isoamylamine, hexylamine, heptylamine, octylamine, nonylamine, decylamine, pentadecylamine, hexadecylamine, heptadecylamine, octadecylamine, methyl ethylamine, methyl isopropylamine, methyl hexylamine, methyl nonylamine, methyl pentadecylamine, methyl octadecylamine, ethyl butylamine, ethyl heptylamine, ethyl octylamine, hexyl heptylamine, hexyl octylamine, dimethylamine, diethylamine, di-n-propylamine, diisopropylamine, di-n-butylamine, di-n-amylamine, diisoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-butenyl-2-amine, n-pentenyl-2-amine, 2,3-dimethylbutenyl-2-amine, dibutenyl-2-amine, n-hexenyl-2-amine, propylenediamine, trimethylamine, triethylamine, tri-n-propylamine, triisopropylamine, tri-n-butylamine, triisobutylamine, tri-sec-butylamine, tri-n-amylamine, methoxyethylamine and ethoxyethylamine; heterocyclic amines such as pyridine, quinoline, isoquinoline, morpholine, piperidine, pyrrolidine, indoline, quinuclidine and azepine; primary arylamines such as anilines, methoxyanilines, ethoxyanilines, o-, m- and p-toluidines, phenylendiamines, benzidines, naphthylamines and o,m,p-chloroanilines; but preferably triethylamines, isopropylamine and diisopropylamine.

Preferred compounds of formula I or salts thereof for use in the novel composition are those wherein R₁ and R₄ are hydrogen. Preferred safeners for these compounds are those safeners of formula II, wherein X is chlorine and R is C₄-C₈alkyl, most preferably 1-methylhexyl.

Particularly preferred novel compositions contain a herbicidally effective sulfonyl urea of formula I, wherein R₁ is hydrogen, R₂ is methyl or methoxy and R₃ is methyl, and R₄ is preferably hydrogen. Particularly suitable safeners for these novel compositions are those safeners of formula II, wherein X is chlorine and R is C₄-C₈alkyl, most preferably 1-methylhexyl.

A very particularly preferred novel composition contains a herbicidally effective amount of a sulfonyl urea of formula Ia 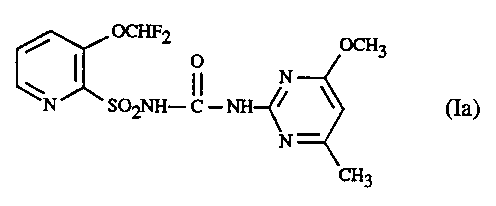
and, as safener, an effective amount of a quinoline derivative of formula IIa 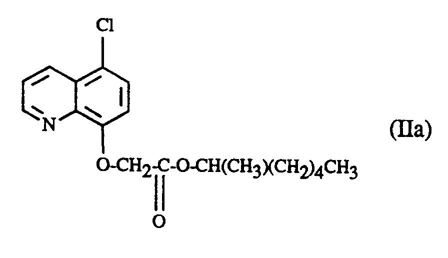
for antagonising the herbicide.

The quinoline derivatives falling within the scope of formula II and their preparation are known and disclosed, inter alia, in EP-A-0 094 349.

The sulfonyl urea herbicides of formula I are disclosed in EP-A-0 103 543 and EP-A-0 582 021. The sulfonyl ureas which are specifically disclosed in EP-A-0 582 021 are distinguished by their particularly good herbicidal activity and may likewise be used in compositions of the invention. The sulfonyl ureas of EP-A-0 582 021 have the formula III 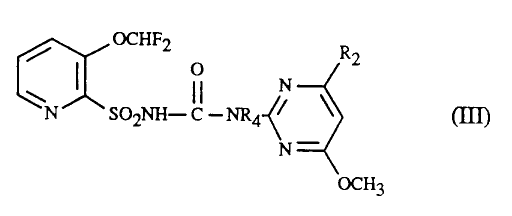
wherein
R₂ is methyl or methoxy, and
R₄ is hydrogen or methyl, and the salts of these compounds with amines, alkali metal bases or alkaline earth metal bases or with quaternary ammonium bases. Among the compounds of formula III, those compounds are preferred in which R₄ is hydrogen.

The compounds of formula I can be prepared by reacting
a) a pyridyl sulfonamide of formula IV 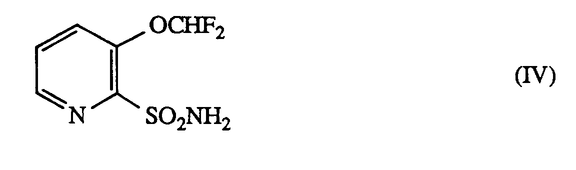 with a N-pyrimidinyl carbamate of formula V 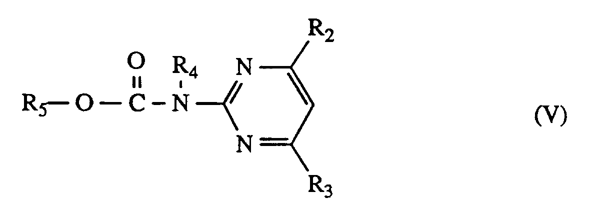 wherein R₂, R₃ and R₄ are as defined for formula I and R₅ is phenyl or 4-tolyl, in the presence of a base, or
b) reacting a pyridyl sulfonamide of formula VI 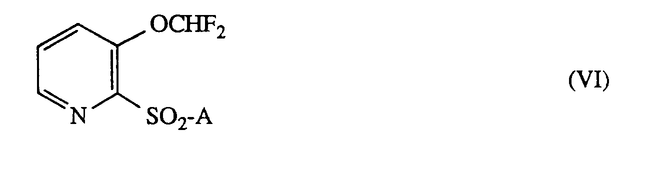 wherein A is 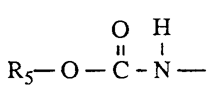 or O=C=N-, wherein R₅ is as defined above, in the presence of a base, with a 2-aminopyrimidine of formula VII 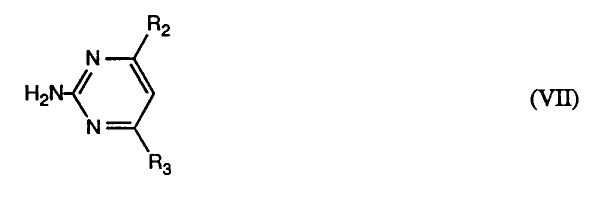 wherein R₂ and R₃ are as defined for formula I, or
c) reacting a pyridyl sulfonamide of formula IV 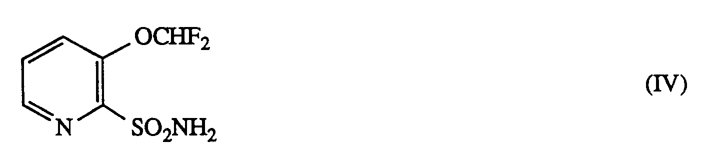 in the presence of a base, with a pyrimidinyl isocyanate of formula VIII 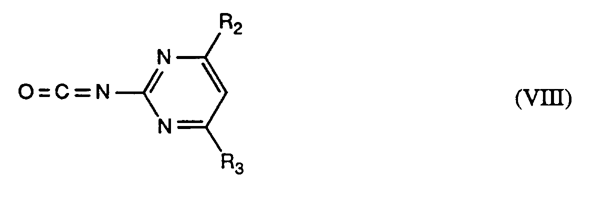 wherein R₂ and R₃ are as defined for formula I, or
d) reacting a compound of formula IX 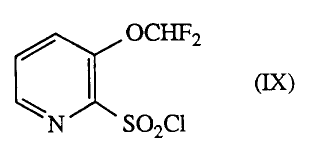 with a compound of formula VII 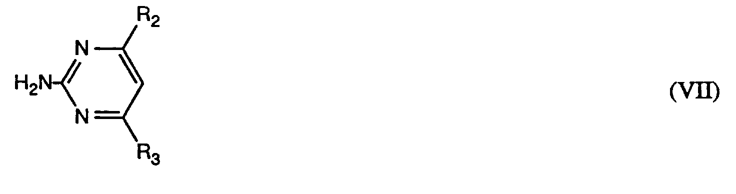 wherein R₂ and R₃ are as defined for formula I, in the presence of an ammonium, phosphonium, sulfonium or alkali metal cyanate salt of formula X

   M⁺OCN⁻ (X)

   wherein M is an alkali metal or the group R₆ R₇ R₈ R₉Q, wherein R₆, R₇, R₈ and R₉ are each independently of one another C₁-C₁₈alkyl, benzyl or phenyl, with the proviso that the total number of carbon atoms is not greater than 36, and Q is nitrogen, sulfur or phosphorus.

The reactions to give the compounds of formula I are conveniently carried out in aprotic, inert, organic solvents. Such solvents are hydrocarbons such as benzene, toluene, xylene or cyclohexane, chlorinated hydrocarbons such as dichloromethane, trichloromethane, tetrachloromethane or chlorobenzene, ethers such as diethyl ether, ethylene glycol dimethyl ether, diethylene glycol dimethyl ether, tetrahydrofuran or dioxane, nitriles such as acetonitrile or propionitrile, amides such as dimethyl formamide, diethyl formamide or N-methylpyrrolidinone. The reaction temperatures are preferably in the range from -20° to +120°C.

The reactions are usually slightly exothermic and can be carried out at room temperature. To shorten the reaction time or to initiate the reaction, it is expedient to heat the reaction mixture for a brief time to the boiling point of the mixture. The reaction times can also be shortened by addition of a few drops of a base as reaction catalyst. Suitable bases are preferably tertiary amines such as trimethylamine, triethylamine, quinuclidine, 1,4-diazabicyclo(2.2.2)octane, 1,5-diazabicyclo(4.3.0)non-5-ene or 1,5-diazabicyclo(5.4.0)undec7-ene. It is, however, also possible to use inorganic bases, including hydrides such as sodium or calcium hydride, hydroxides such as sodium and potassium hydroxide, carbonates such as sodium and potassium carbonate or hydrogencarbonates such as potassium and sodium hydrogencarbonate.

The final products of formula I can be isolated by concentration and/or evaporation of the solvent and purified by recrystallisation or trituration of the solid residue in solvents in which they are not readily soluble, typically in ethers, aromatic hydrocarbons or chlorinated hydrocarbons.

The compounds of formulae IV, V, VI, VII, VIII, IX and X are either known or can be prepared by methods analogous to known ones. Process variant a) is disclosed, inter alia, in EP-A-0 103 543, and process variant b) in EP-A-0 101 670. Process variants c) and d) are disclosed in EP-A-0 459 949 and Swiss patent 662 348.

The invention further relates to a method of selectively controlling weeds in crops of cultivated plants, which comprises treating cultivated plants, the seeds or seedlings or the crop area thereof, simultaneously or independently of one another, with a herbicidally effective amount of the sulfonyl urea of formula I and an effective amount of a quinoline derivative of formula II as safener for antagonising the herbicide.

Suitable crop plants which can be protected by the quinoline derivatives of formula II against the harmful action of the aforementioned herbicides are preferably those that are important in the food or textile sector, typically sugar cane and, in particular, millet, maize, rice and other cereals (wheat, rye, barley, oats), most preferably wheat and barley.

The weeds to be controlled may be monocot as well as dicot weeds.

Crop plants or parts of said plants are typically those referred to above. Crop areas are the areas already under cultivation with the crop plants or seeds thereof, as well as the areas intended for cultivation with said crop plants.

Depending on the end use, the safener or antidote of formula II can be used for pretreating seeds of the crop plants (dressing of seeds of seedlings) or it can be added to the soil before or after sowing. It can, however, also be applied pre- or postemergence by itself alone or together with the herbicide. The treatment of the plant or seeds with the safener can therefore in principle be carried out irrespective of the time of application of the phytotoxic chemical. It can, however, also be carried out by simultaneous application of the phytotoxic chemical and safener (tank mixture). The pre-emergence treatment includes treatment of the crop area before sowing as well as treatment of the crop area after sowing but before emergence of the plants.

The concentration of safener with respect to the herbicide will depend substantially on the mode of application. Where a field treatment is carried out either by using a tank mixture with a combination of safener and herbicide or by separate application of safener and herbicide, the ratio of safener to herbicide is normally from 1:100 to 10:1, prefeerably from 1:20 to 10:1.

In field treatment it is usual to apply 0.001 to 5.0 kg/ha, preferably 0.01 to 0.5 kg/ha, of safener.

The concentration of herbicide is normally in the range from 0.001 to 2 kg/ha, but is preferably from 0.05 to 1 kg/ha.

For seed dressing, 0.001 to 10 g of safener/kg of seeds, preferably 0.05 to 2 g of safener/kg of seeds, is normally applied. If the safener is used in liquid form shortly before sowing with seed swelling, then it is expedient to use safener solutions which contain the active ingredient in a concentration of 1 to 10 000 ppm, preferably of 100 to 1000 ppm.

For application, the compounds of formula II or combinations of compounds of formula II with the herbicides to be antagonised are conveniently used together with the adjuvants conventionally employed in the art of formulation, and are therefore formulated in known manner to emulsifiable concentrates, coatable pastes, directly sprayable or dilutable solutions, dilute emulsions, wettable powders, soluble powders, dusts, granulates, and also encapsulations in e.g. polymer substances. As with the compositions, the methods of application such as spraying, atomising, dusting, scattering or pouring, are chosen in accordance with the intended objectives and the prevailing circumstances.

The formulations, i.e. the compositions, preparations or mixtures containing the compound (active ingredient) of formula II or a combination of formula II with the herbicide of formula I to be antagonised and, where appropriate, a solid or liquid adjuvant, are prepared in known manner, e.g. by homogeneously mixing and/or grinding the active ingredients with extenders, e.g. solvents, solid carriers and, in some cases, surface-active compounds (surfactants).

Suitable solvents are: aromatic hydrocarbons, preferably the fractions containing 8 to 12 carbon atoms such as xylene mixtures or substituted naphthalenes; phthalates such as dibutyl or dioctyl phthalate; aliphatic hydrocarbons such as cyclohexane or paraffins; alcohols ad glycols and their ethers and esters such as ethanol, ethylene glycol, ethylene glycol monomethyl or monoethyl ether; ketones such as cyclohexanone; strongly polar solvents such as N-methyl-2-pyrrolidone, dimethyl sulfoxide or dimethyl formamide; as well as vegetable oils or epoxidised vegetable oils such as epoxidised coconut oil or soybean oil; or water.

The solid carriers used typically for dusts and dispersible powders are normally natural mineral fillers such as calcite, talcum, kaolin, montmorillonite or attapulgite. To improve the physical properties it is also possible to add highly dispersed silicic acid or highly dispersed absorbent polymers. Suitable granulated adsorptive carriers are porous types, conveniently pumice, broken brick, sepiolite or bentonite; and suitable nonsorbent carriers are materials such as calcite or sand. In addition, innumerable pregranulated materials of inorganic or organic nature can be used, especially dolomite or pulverised plant residues.

Depending on the nature of the compound of formula II to be formulated and, where appropriate, also on the herbicide of formula I to be antagonised, suitable surface-active compounds are nonionic, cationic and/or anionic surfactants having good emulsifying, dispersing and wetting properties. The term "surfactants" will also be understood as comprising mixtures of surfactants.

Suitable anionic surfactants can be both water-soluble soaps and water-soluble synthetic surface-active compounds.

Suitable soaps are the alkali metal salts, alkaline earth metal salts or unsubstituted or substituted ammonium salts of higher fatty acids (C₁₀-C₂₂), e.g. the sodium or potassium salts of oleic or stearic acid, or of natural fatty acid mixtures which can be obtained, inter alia from coconut oil or tallow oil. Further suitable surfactants are also the fatty acid methyltaurin salts.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammonium salts and generally contain a C₈-C₂₂-alkyl radical which also includes the alkyl moiety of acyl radicals, e.g. the sodium or calcium salt of ligninsulfonic acid, of dodecylsulfate, or of a mixture of fatty alcohol sulfates obtained from natural fatty acids. These compounds also comprise the salts of sulfated and sulfonated fatty alcohol/ethylene oxide adducts. The sulfonated benzimidazole derivatives preferably contain 2 sulfonic acid groups and one fatty acid radical containing about 8 to 22 carbon atoms. Examples of alkylarylsulfonates are the sodium, calcium or triethanolamine salts of dodecylbenzenesulfonic acid, dibutylnaphthalenesulfonic acid, or of a condensate of naphthalenesulfonic acid and formaldehyde. Also suitable are corresponding phosphates, e.g. salts of the phosphated polyadduct of p-nonylphenol with 4 to 14 mol of ethylene oxide.

Nonionic surfactants are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols. Further suitable nonionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of nonionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, e.g. polyoxyethylene sorbitan trioleate, are also suitable nonionic surfactants.

Cationic surfactants are preferably quaternary ammonium salts carrying, as N-substituent, at least one C₈-C₂₂alkyl radical and, as further substituents, unsubstituted or halogenated lower alkyl, benzyl or hydroxy-lower alkyl radicals. The salts are preferably in the form of halides, methylsulfates or ethylsulfates, e.g. stearyltrimethylammonium chloride or benzyl bis(2-chloroethyl)ethylammonium bromide.

The surfactants customarily employed in the art of formulation are described e.g. in the following publications:
- "Mc Cutcheon's Detergents and Emulsifiers Annual", Mc Publishing Corp., Glen Rock, New Jersey, 1988.
- Dr. Helmut Stache "Tensid-Taschenbuch" (Handbook of Surfactants), Carl Hanser Verlag, Munich/Vienna 1981.

The agrochemical compositions usually contain 0.1 to 99% by weight, preferably 0.1 to 95 % by weight, of a compound of formula II or mixture of safener/herbicide, 1 to 99.9 % by weight of a solid or liquid adjuvant, and 0 to 25 % by weight, preferably 0.1 to 25 % by weight, of a surfactant.

Whereas commercial products will preferably be formulated as concentrates, the end user will normally use dilute formulations.

The compositions may also contain further ingredients such as stabilisers, antifoams, viscosity regulators, binders, tackifiers as well as fertilisers or other chemical agents to obtain special effects.

Different methods and techniques may suitably be used for applying the compounds of formula II or compositions containing them for protecting cultivated plants from the harmful effects of herbicides of formula I, typically the following:

### i) Seed dressing

a) Dressing the seeds with a wettable powder formulation of the compound of formula II by shaking in a vessel to give a homogeneous distribution on the surface of the seeds (dry treatment), using up to c. 1 to 500 g of compound of formula II (4 g to 2 g of wettable powder) per 100 kg of seeds.
b) Dressing seeds with an emulsifiable concentrate of the compound of formula II by method a) (wet treatment).
c) Dressing by immersing the seeds in a mixture containing 100-1000 ppm of compound of formula II for 1 to 72 hours and, if desired, subsequently drying the seeds (seed soaking).

Normally 1 to 1000 g, preferably 5 to 250 g, of safener is used per 100 kg of seeds. However, depending on the method employed, which also permits the use of other chemicals or micronutrients, the indicated limiting concentrations may be increased or lowered (repeat dressing).

### ii) Application from a tank mixture

A liquid formulation of a mixture of safener and herbicide (reciprocal ratio from 10:1 to 1:100) is used, the concentration of herbicide being from 0.01 to 5.0 kg/ha. This tank mixture is applied before or after sowing.

### iii) Application in the furrow

The safener formulated as emulsifiable concentrate, wettable powder or granulate is applied to the open furrow in which the seeds have been sown. After covering the furrow in the usual manner, the herbicide is applied pre-emergence.

### iv) Controlled release of safener

A solution of the compound of formula II is applied to mineral granulate substrates or polymerised granulates (urea/formaldehyde) and allowed to dry. If desired, a coating can be applied (coated granulates) which permits the growth regulator to be released over a specific period of time.

The preparation of compounds of formula I is illustrated in more detail by the following Examples.

### Example P1: Preparation of 2.isopropylthio-3-hydroxypyridine

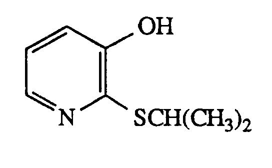

14 g of potassium tert-butylate are added in increments at a temperature of +5°C to a solution of 16.4 g of 2-mercapto-3-hydroxypyridine (known from Tetrahedron **21,**2191, (1980)) in 155 ml of dimethyl formamide. Then 12.5 ml of isopropyl iodide are added dropwise at a temperature of 0 to +2°C over 15 minutes. After stirring for 2 hours at room temperature, the reaction mixture is extracted with 400 ml of ice-water and 200 ml of ethyl acetate. After adjustment of the pH to 7 to 8 with 2N hydrochloric acid, the aqueous phase is washed with 4 x 150 ml of ethyl acetate. The combined organic phases are washed with 3 x 100 ml of water, concentrated to a volume of 100 ml under vacuum and extracted with 4 x 50 ml of 2N aqueous sodium hydroxide solution at a temperature of 0°C. The aqueous phase is then adjusted with 2N hydrochloric acid to pH 7-8 and extracted with 4 x 50 ml of ethyl acetate. The organic phase is dried, filtered over 100 g of silica gel, and the filtrate is washed with 4 x 50 ml of ethyl acetate and concentrated by evaporation, giving a crystalline residue which is triturated with petroleum ether and then isolated by filtration. Yield: 14.6 g of 2-isopropylthio-3-hydroxypyridine with a melting point of 64°C.

### Example P2: Preparation of 2-isopropylthio-3-fluoromethoxypyridine:

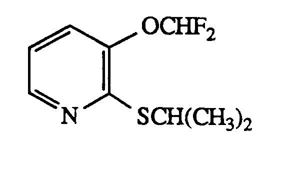

464 ml of a 30% aqueous solution of sodium hydroxide are added dropwise over 15 minutes to a solution of 118.4 g of 2-isopropylthio-3-hydroxypyridine in 560 ml of dDioxane. Then 121 g of freon-22 are introduced at a temperature of 80°C over 2 hours and the reaction mixture is stirred for another 90 minutes. The reaction mixture is cooled to room temperature and then extracted with a mixture of 2500 ml of ice-water and 1000 ml of methylene chloride. The phases are washed with 4 x 50 ml of methylene chloride and once with 100 ml of ice-water. The combined organic phases are then dried over sodium sulfate and concentrated under vacuum. Purification by chromatography over silica gel with ethyl acetate/n-hexane 1:9 as eluant gives 105.7 g of 2-isopropylthio-3-difluoromethoxypyridine in the form of an oil with n_{D}²¹= 1.5088.

### Example P3: Preparation of 3-difluoromethoxypyridin-2-yl-sulfonamide:

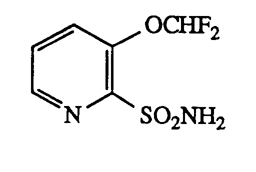

142 g of chlorine gas are passed into a mixture of 105.7 g 2-isopropylthio-3-difluoromethoxypyridine, 1000 ml of dichloromethane and 1723 ml of 1N hydrochloric acid over 50 minutes at a temperature of -5 to 0°C. Alter stirring for 20 minutes at a temperature of -30°C, nitrogen is introduced over 15 minutes. The phases are washed with 3 x 250 ml of ice-water and 250 ml of dichloromethane, and the combined organic phases are dried over sodium sulfate. This reaction mixture is then added dropwise at a temperature of -50 to -40°C over 40 minutes to a mixture of 122.7 g of ammonia in 250 ml in dichloromethane. After stirring for 15 hours and subsequent filtration, the reaction mixture is concentrated by evaporation, the residue is triturated with petroleum ether, and the resulting crystal agglomerate is isolated by filtration and dried, giving 85.9 g of 3-difluoromethoxypyridin-2-yl-sulfonamide with a melting point of 85-86°C.

### Example P4: Preparation of N-(3-difluoromethoxypyridin-2-yl-sulfonyl)-N'-(4-methyl-6-methoxypyrimidin-2-yl)urea (compound 1.03):

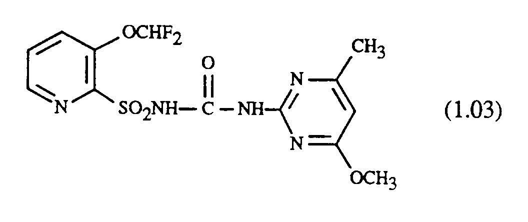

4.97 g of N-(4-methyl-6-methoxypyrimidin-2-yl)phenylcarbamate and 2.95 ml of 1,5-diazabicyclo[5.4.0]undec-5-ene are added in succession to a solution of 4.03 g of difluoromethoxypyridin-2-yl-sulfonamide in 40 ml of acetonitrile. After stirring for 60 minutes, the reaction mixture is concentrated under vacuum. Then the oily residue is triturated with 12 ml of 2N hydrochloric acid and diluted with 10 ml of water. The crystallised product is isolated by filtration and washed in succession with water and diethyl ether, giving N-(3-difluoromethoxypyridin-2-yl-sulfonyl)-N'-(4-methyl-6-methoxypyrimidin-2-yl)urea (compound 1.03) with a melting point of 151-154 °C.

The compounds of formula I listed in Table 1 and their intermediates are prepared in analogous manner.

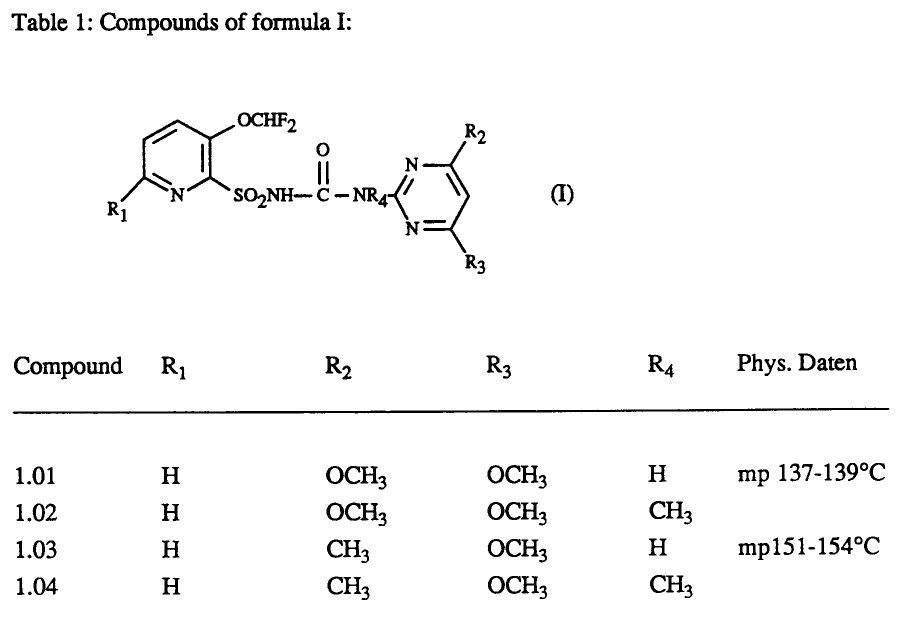

### Formulation Examples for mixtures of formula I and the safener of formula II (throughout percentages are by weight)

| F1. Solutions | a) | b) | c) | d) |
|---|---|---|---|---|
| compound mixture | 5 % | 10 % | 50 % | 90 % |
| dipropylene glycol methyl ether | - | 20 % | 20 % | - |
| polyethylene glycol MG 400 | 20 % | 10 % | - | - |
| N-methyl-2-pyrrolidone | - | - | 30 % | 10 % |
| mixture of aromatic hydrocarbons C₉-C₁₂ | 75 % | 60 % | - | - |

The solutions are suitable for use as microdrops.

| F2. Wettable powders | a) | b) | c) | d) |
|---|---|---|---|---|
| compound mixture | 5 % | 25 % | 50 % | 80 % |
| sodium ligninsulfonate | 4 % | - | 3 % | - |
| sodium laurylsulfate | 2 % | 3 % | - | 4 % |
| sodium diisobutylnaphthalene sulfonate | - | 6 % | 5 % | 6 % |
| octylphenol polyglycol ether (7-8 mol EO) | - | 1 % | 2 % | - |
| highly dispersed silica | 1 % | 3 % | 5 % | 10 % |
| kaolin | 88 % | 62 % | 35 % | - |

The active ingredient is throughly mixed with the adjuvants and the mixture is ground in a suitable mill to give wettable powders which can be diluted with water to give suspensions of any desired concentration.

| F3. Coated granulates | a) | b) | c) |
|---|---|---|---|
| compound mixture | 0.1 % | 5 % | 15 % |
| highly dispersed silica | 0.9 % | 2 % | 2 % |
| inorg. carrier (⌀ 0.1-1 mm) e.g. CaCO₃ or SiO₂ | 99.0 % | 93 % | 83 % |

The active ingredient is dissolved in methylene chloride, the solution is sprayed on to the carrier, and the solvent is removed under vacuum.

| F4. Coated granulates | a) | b) | c) |
|---|---|---|---|
| compound mixture | 0.1 % | 5 % | 15 % |
| polyethylene glycol MG 200 | 1.0 % | 2 % | 3 % |
| highly dispersed silica | 0.9 % | 1 % | 2 % |
| inorg. carrier (⌀ 0.1 - 1 mm) e.g. CaCO₃ or SiO₂ | 98.0 % | 92 % | 80 % |

The finely ground active ingredient is uniformly applied in a mixer to the kaolin moistened with polyethylene glycol. Non-dusty coated granulates are obtained in this manner.

| F5. Extruder granulates | a) | b) | c) | d) |
|---|---|---|---|---|
| compound mixture | 0.1 % | 3 % | 5 % | 15 % |
| sodium ligninsulfonate | 1.5 % | 2 % | 3 % | 4 % |
| carboxymethyl cellulose | 1.4 % | 2 % | 2 % | 2 % |
| kaolin | 97.0 % | 93 % | 90 % | 79 % |

The active ingredient is mixed with the adjuvants and the mixture is moistened with water. This mixture is extruded and then dried in a stream of air.

| F6. Dusts | a) | b) | c) |
|---|---|---|---|
| compound mixture | 0.1 % | 1 % | 5 % |
| talcum | 39.9 % | 49 % | 35 % |
| kaolin | 60.0 % | 50 % | 60 % |

Ready for use dusts are obtained by mixing the the active ingredient with the carriers on a suitable mill.

| F7. Suspension concentrates | a) | b) | c) | d) |
|---|---|---|---|---|
| compound mixture | 3 % | 10 % | 25 % | 50 % |
| ethylene glycol | 5 % | 5 % | 5 % | 5 % |
| nonylphenol polyglycol ether (15 mol EO) | - | 1 % | 2 % | - |
| sodium ligninsulfonate | 3 % | 3 % | 4 % | 5 % |
| carboxymethyl cellulose | 1 % | 1 % | 1 % | 1 % |
| 37% aqueous formaldehyde solution | 0.2 % | 0.2 % | 0.2 % | 0.2 % |
| silicone oil emulsion | 0.8 % | 0.8 % | 0.8 % | 0.8 % |
| water | 87 % | 79 % | 62 % | 38 % |

The finely ground active ingredient is intimately mixed with the adjuvants to give a suspension concentrate from which suspensions of any desired concentration can be prepared by dilution with water.

### Biological Examples

### Example B1: Postermergence phytotoxic action of the herbicides of formula I and of the mixtures of herbicide/safener of formula II on wheat

Under greenhouse conditions, wheat is cultivated in plastic pots to the 4-leaf stage. In this stage, the herbicide of Table 1 alone as well as the mixtures of the herbicide with the safener 1-methyl-1-hex-1-yl 2-(5-chloro-8-quinolinoxy)acetate are applied to the test plants. Application is made in the form of an aqueous suspension of the test compounds according to Example P7 in 500 l of water/ha. Evaluation (in %) is made 28 days after application according to the following rating:
100 % = test plant withered
0 % = no phytotoxic action.

The results are set forth in Table B1. These results show that the damage caused by the safener 1-methyl-1-hex-1-yl 2-(5-chloro-8-quinolinoxy)acetate to wheat and barley can be markedly reduced.

**Table B1**

| The concentration of the safener 1-methyl-1-hex-1-yl 2-(5-chloro-8-quinolinoxy)acetate in the following Table is 125 g a.i./ha. | | | |
|---|---|---|---|
| Herbicide Compound | Concentration of herbicide | Safener | Phytotoxic action on wheat (%) |
| 1.01 | 30 g/ha | none | 40 |
| 1.01 | 30 g/ha | with | 0 |
| 1.01 | 15 g/ha | none | 15 |
| 1.01 | 15 g/ha | with | 0 |
| 1.03 | 30 g/ha | none | 80 |
| 1.03 | 30 g/ha | with | 30 |
| 1.03 | 15 g/ha | none | 25 |
| 1.03 | 15 g/ha | with | 5 |

The same results are obtained by formulating the compounds of formula I and/or II according to Examples F1 to F6.

## Claims

1. A selective herbicidal composition which contains, in addition to inert components such as carriers, solvents and wetting agents, a mixture comprising
a) a herbicidally effective amount of a sulfonyl urea of formula I 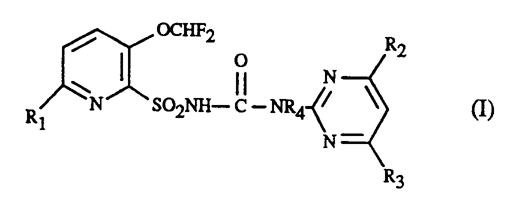 wherein
R₁ is hydrogen or methyl;
R₂ and R₃ are each independently of the other methyl or methoxy; and
R₄ is hydrogen or methyl, or a salt of this compound with an amine, an alkali metal or alkaline earth metal base or with a quaternary ammonium base, as active component; and
b) an effective amount of a quinoline derivative of formula II 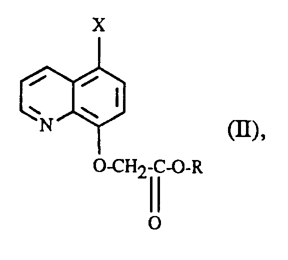 wherein
R is hydrogen or C₁-₈alkyl, and
X is hydrogen or chlorine,
as safener for antagonising the herbicide.

2. A composition according to claim 1, wherein R₁ is hydrogen.

3. A composition according to claim 2, wherein R₄ is hydrogen.

4. A composition according to claim 1, wherein X is chlorine and R is C₄-C₈alkyl.

5. A composition according to claim 4, wherein R is 1-methylhexyl.

6. A composition according to claim 1, wherein R₁ is hydrogen, R₂ is methyl or methoxy and R₃ is methoxy.

7. A composition according to claim 6, wherein R₄ is hydrogen.

8. A composition according to claim 6, wherein X is chlorine and R is C₄-C₈alkyl.

9. A composition according to claim 6, wherein R is 1-methylhexyl.

10. A composition according to claim 1, which comprises a herbicidally effective amount of a sulfonyl urea of formula Ia 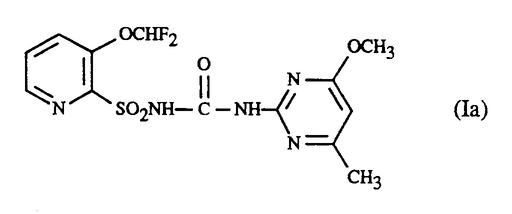 and an effective amount of a quinoline derivative of formula IIa 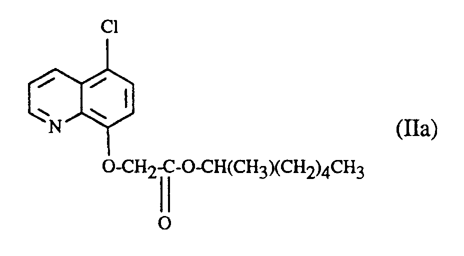 as safener for antagonising the herbicide.

11. A method of selectively controlling weeds in crops of cultivated plants, which comprises treating said plants, the seeds or crop area thereof, simultaneously or independently of one another, with a herbicidally effective amount of the sulfonyl urea of formula I and an effective amount of a quinoline derivative of formula II as safener for antagonising the herbicide.

12. A method according to claim 11, which comprises treating crops of cultivated plants or areas for cropping with cultivated plants, with 0.05 to 2 kg/ha of a compound of formula I according to claim 1 and an amount of 0.01 to 0.5 kg/ha of a compound of formula II according to claim 1.

13. A method according to claim 11 for selectively controlling weeds and grasses in cereals crops.

## Patentansprüche

1. Selektive herbizide Zusammensetzung, die neben inerten Komponenten, wie Trägerstoffen, Lösungsmitteln und Benetzungsmitteln, ein Gemisch enthält, umfassend
a) als Wirkstoff eine herbizid wirksame Menge eines Sulfonylharnstoffs der Formel I 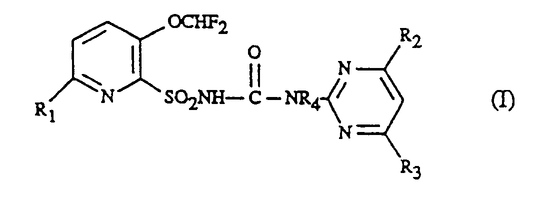 worin
R₁ Wasserstoff oder Methyl bedeutet;
R₂ und R₃ unabhängig voneinander Methyl oder Methoxy bedeuten; und
R₄ Wasserstoff oder Methyl bedeutet oder ein Salz dieser Verbindung mit einem Amin, einer Alkalimetall- oder Erdalkalimetallbase oder mit einer quaternären Ammoniumbase; und
b) als Safener, der antagonistisch zum Herbizid wirkt, eine wirksame Menge eines Chinolinderivats der Formel II 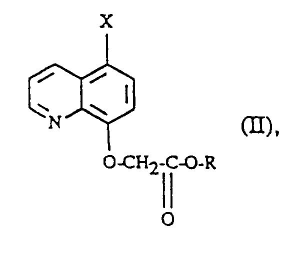 worin
R Wasserstoff oder C₁-C₈-Alkyl darstellt und
X Wasserstoff oder Chlor darstellt.

2. Zusammensetzung gemäß Anspruch 1, worin R₁ Wasserstoff bedeutet.

3. Zusammensetzung gemäß Anspruch 2, worin R₄ Wasserstoff bedeutet.

4. Zusammensetzung gemäß Anspruch 1, worin X Chlor darstellt und R C₄-C₈-Alkyl darstellt.

5. Zusammensetzung gemäß Anspruch 4, worin R 1-Methylhexyl bedeutet.

6. Zusammensetzung gemäß Anspruch 1, worin R₁ Wasserstoff bedeutet, R₂ Methyl oder Methoxy bedeutet und R₃ Methoxy bedeutet.

7. Zusammensetzung gemäß Anspruch 6, worin R₄ Wasserstoff bedeutet.

8. Zusammensetzung gemäß Anspruch 6, worin X Chlor darstellt und R C₄-C₈-Alkyl bedeutet.

9. Zusammensetzung gemäß Anspruch 6, worin R 1-Methylhexyl darstellt.

10. Zusammensetzung gemäß Anspruch 1, umfassend eine herbizid wirksame Menge eines Sulfonylharnstoffs der Formel Ia 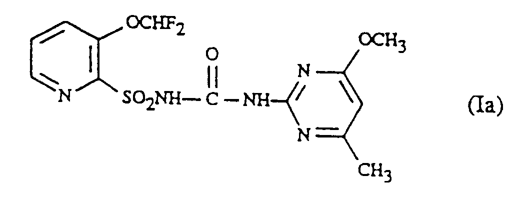 und als Safener, der antagonistisch zum Herbizid wirkt, eine wirksame Menge eines Chinolinderivates der Formel IIa

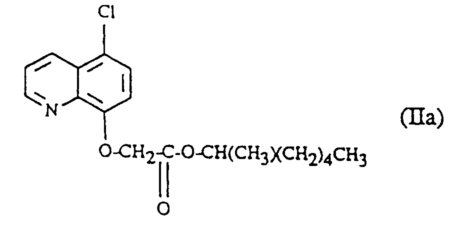

11. Verfahren zum selektiven Bekämpfen von Unkräutern in Nutzpflanzenkulturen, umfassend Behandeln der Pflanzen, des Saatguts oder deren Anbaufläche gleichzeitig oder unabhängig voneinander mit einer herbizid wirksamen Menge des Sulfonylharnstoffs der Formel I und einer als Safener, der antagonistisch zum Herbizid wirkt, wirksamen Menge eines Chinolinderivats der Formel II.

12. Verfahren gemäß Anspruch 11, umfassend Behandeln der Nutzpflanzenkulturen oder Anbauflächen für Nutzpflanzen mit 0,05 bis 2 kg/ha einer Verbindung der Formel I gemäß Anspruch 1 und einer Menge von 0,01 bis 0,5 kg/ha einer Verbindung der Formel II gemäß Anspruch 1.

13. Verfahren gemäß Anspruch 11 zum selektiven Bekämpfen von Unkräutern und Gräsern in Getreidekulturen.

## Revendications

1. Composition herbicide sélective contenant, avec des composants inertes tels que des véhicules, des solvants et des agents mouillants, un mélange consistant en
a) une quantité herbicide efficace d'une sulfonylurée de formule I 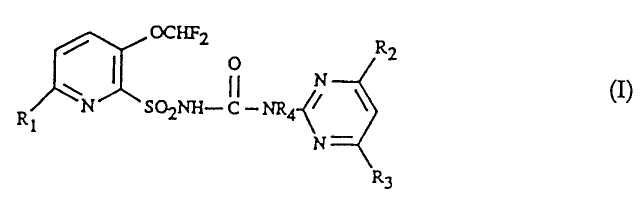 dans laquelle
R₁ représente l'hydrogène ou un groupe méthyle ;
R₂ et R₃ représentent chacun, indépendamment l'un de l'autre, un groupe méthyle ou méthoxy ; et
R₄ représente l'hydrogène ou un groupe méthyle, ou d'un sel d'un tel composé et d'une amine, d'une base de métal alcalin ou alcalinoterreux ou d'une base d'ammonium quaternaire, en tant que composant actif ; et
b) une quantité efficace d'un dérivé de quinoléine de formule II 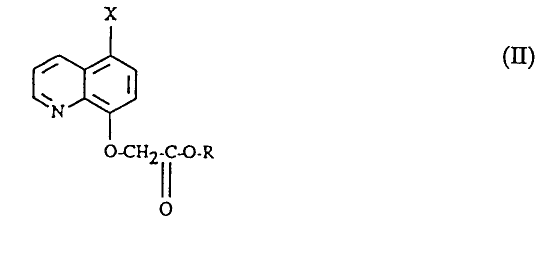 dans laquelle
R représente l'hydrogène ou un groupe alkyle en C₁-C₈ et
X représente l'hydrogène ou le chlore,
en tant qu'antidote antagonisant l'herbicide.

2. Une composition selon revendication 1, pour laquelle R₁ représente l'hydrogène.

3. Une composition selon revendication 2, pour laquelle R₄ représente l'hydrogène.

4. Une composition selon revendication 1, pour laquelle X représente le chlore et R un groupe alkyle en C₄-C₈.

5. Une composition selon revendication 4, pour laquelle R représente un groupe 1-méthylhexyle.

6. Une composition selon revendication 1, pour laquelle R₁ représente l'hydrogène, R₂ un groupe méthyle ou méthoxy et R₃ un groupe méthoxy.

7. Une composition selon revendication 6, pour laquelle R₄ représente l'hydrogène.

8. Une composition selon revendication 6, pour laquelle X représente le chlore et R un groupe alkyle en C₄-C₈.

9. Une composition selon revendication 6, pour laquelle R représente un groupe 1-méthylhexyle.

10. Une composition selon revendication 1, qui contient une quantité herbicide efficace d'une sulfonylurée de formule Ia 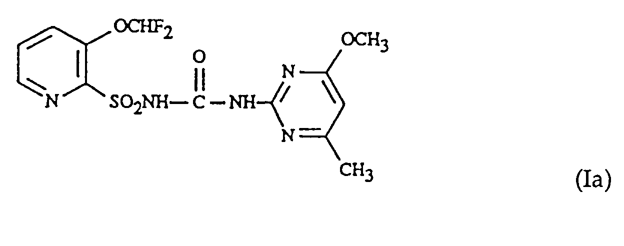 et une quantité efficace d'un dérivé de la quinoléine de formule IIa 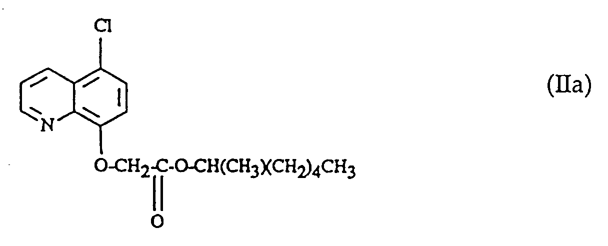 en tant qu'antidote antagonisant l'herbicide.

11. Un procédé pour combattre sélectivement les végétaux adventices dans les cultures de végétaux utiles, procédé qui consiste à traiter les végétaux, leurs semences ou leur aire de culture, simultanément ou indépendamment par une quantité herbicide efficace de la sulfonylurée de formule I et une quantité efficace d'un dérivé de la quinoléine de formule II en tant qu'antidote antagonisant l'herbicide.

12. Un procédé selon revendication 11, qui consiste à traiter les cultures de la plante utile ou les aires prévues pour leur culture par 0,05 à 2 kg/ha d'un composé de formule I selon revendication 1 et 0,01 à 0,5 kg/ha d'un composé de formule II selon revendication 1.

13. Un procédé selon revendication 11 pour combattre sélectivement les végétaux adventices dans les cultures de céréales.
